# EUROPEAN PATENT SPECIFICATION

(11) **EP 4 258 999 B1**
(45) Date of publication and mention of the grant of the patent: **23.04.2025**
(21) Application number: 21819529.5
(22) Date of filing: 10.12.2021
(51) Int. Cl.: A61B 8/08, A61B 10/04, A61B 34/10, G01S 5/18, A61B 90/00, A61B 34/30, A61B 34/20, A61B 17/00, A61B 8/00, A61B 5/00, A61B 34/00, G01S 7/52, G01S 15/66, G01S 15/89

(54) **MICRO-DEVICE TRACKING AND VIZUALISATION SYSTEM**
MICRO-APPARAT-VERFOLGUNGS- UND -BILDGEBUNGSSYSTEM
SYSTEM DE SUIVI PAR UN MICRO-DISPOSITIV ET SYSTEME D'IMAGERIE

(30) Priority: 11.12.2020 EP 20306554
(43) Date of publication of application: 18.10.2023
(73) Proprietor: Robeaute, 75002 Paris (FR); Sorbonne Université, 75006 Paris (FR); Centre National de la Recherche Scientifique, 75016 Paris (FR); Institut National de la Santé et de la Recherche Médicale (INSERM), 75013 Paris (FR)
(72) Inventor: FRANCOIS, Quentin, 75002 Paris (FR); DUPLAT, Bertrand, 75002 Paris (FR); HALIYO, Sinan, 75006 Paris (FR); RÉGNIER, Stéphane, 75006 Paris (FR); ZARADER, Pierre, 75002 Paris (FR); COUTURE, Olivier, 75016 Paris (FR); COUDERT, Antoine, 75016 Paris (FR)
(74) Representative: Icosa
(86) International application number: PCT/EP2021/085202
(87) International publication number: WO 2022/123013

(56) References cited:
- WO-A1-2020/135945
- US-A1- 2014 094 695
- US-A1- 2016 058 517

## Description

### FIELD OF INVENTION

The present invention relates to ultrasound positioning and imaging used in device localization inside a target body structure of a patient, by means of the same probe.

### BACKGROUND OF INVENTION

For some advanced medical treatments, it might be relevant to use microrobots inserted in a target body part of a patient in order, for example, to deliver a very precise amount of drug to a very precise point. For safety purposes, this microrobot should be as autonomous as possible and most preferably controlled from outside the patient's body in a contactless manner. This micro-device thus needs a wireless localization system comprising an internal referential, in order to be tracked and precisely located while moving inside the target body part. This system should also be able, in order to enable a surgeon to be in full control of the situation, to offer a precise 3D visualization of the localization of said micro-robot inside the target body part.

There is a need for improving the tracking system and especially the localization for this kind of microrobots in correspondence to the anatomy of the target body part. The position of the microrobot with respect to anatomic features, such as functional areas, vessels or nerve, is of primary importance since it will define the path and target point of the robot, inside the target body part. A 3D imaging modality is thus necessary to envision these features and permit path planning. Moreover, the imaging modality and the microrobot positioning has to be perfectly co-registered with a precision better than 1 mm. It is thus important for the imaging system and the positioning system to be performed in the same internal referential, ideally through the same technique.

Additionally, regarding the specific case of the brain, even if the brain is encapsulated by the skull, it can move, distort, expand or dilate. The micro-robot itself might modify the surrounding anatomy while it moves through tissue. Tissue physiology such as blood flow might be impacted by the micro-robot and its action. Consequently, it would be ideal to provide a frequent refreshment of the images, along with the position of the microrobot, in order to have an up-to-date spatial information of the microrobot localized in the referential space. A commonly known wireless communication path between two elements is ultrasound communication. Such technique could be used for positioning the robot. A commonly known way to obtain reliable 3D body imagery is also ultrasound imaging. Both approaches could therefore be performed in alternance with a similar or identical apparatus.

There are several implementations for 3D ultrasound imaging, Brightness-mode, elastography or Doppler. One of the possibilities for highly precise imaging of the vascular network is ultrasound localization microscopy (ULM). The core idea of the ULM is generally known to be a very highly precise ultrasound imagery method based on the introduction of sparse punctual sources in the medium being imaged, to highlight specific parts. These sources are usually air microbubbles, more precisely millions of microbubbles, also called contrast agents. In order to obtain an ULM image of a target body structure like, for example, the brain vascular system, microbubbles are injected in the patient. Many 3D transcranial images are acquired. Microbubbles are localized and within a few minutes, a 3D ULM image is obtained. Thanks to these microbubbles, the vascular system is resolved under the diffraction barrier (the precision reaching a λ/10 precision). A super-resolved image is thus constructed by localizing each bubble center separately and accumulating their positions to recover the vessel's network, several times smaller than the wavelength. The use of microbubbles (with a diameter ranging from 1 to 3µm), thanks to their high deformation, allows the imaging system to outperform accuracy limitations due to the classical wave diffraction theory which is around half of the wavelength and to bypass the usual compromise to be found between wave penetration (favorized in the low wave frequency range) and image resolution (favorized in the high wave frequency range). This enables to visualize details which remain invisible on images built by conventional echography, Doppler echography in particular. In particular regarding brain vascularization, this technology enables the creation of highly precise images enabling a precise 3D mapping of a patient's brain vascular system.

Approaches proposed in ULM can also be implemented for improving the positioning of the microrobot with ultrasound. Localization of microbubbles in ULM is not limited in resolution by the wavelength, but rather by the signal-to-noise ratio (SNR) linked to the detection of the microbubbles. A similar idea can be implemented for the localization of the robot, which could yield very high SNR and, hence, allow very precise localization. This precision could be well below 100 micrometers for frequencies that are capable of penetrating the skull (<3 MHz).

WO2020135945A1 relates to a system and a method for real-time localization of a millimetric or submillimetric object in a viscoelastic medium, and employs ultrasound signals.

The present invention aims at solving the visualization and tracking precision issue in co-registering the ultrasound signals used either to acquire a 3D image of the target body part or to track the micro-device.

The invention is defined in claim 1, with further aspects in the dependent claims.

### SUMMARY OF THE DISCLOSURE

This disclosure thus relates to a micro-device tracking and visualization system configured to monitor a target body part of a patient and localizing a micro-device inside said target body part, the tracking system comprising:
- a micro-device designed to be remotely steered and controlled from outside the target body part,
- a control unit comprising a memory, the memory being configured to store at least one ultrasound image of the target body part,
- at least one probe configured to be brought in contact with a securing body part of the patient, the securing body part surrounding at least partially the target body part,
- at least one tracker configured to be connected to the micro-device,
- at least a screen,
   wherein the at least one probe and the at least one tracker communicate by means of ultrasound technology, the control unit being thus able to localize, in real time, the at least one tracker inside the target body part within an internal referential defined with regards to the at least one probe,
   wherein the control unit is further designed to display, on the screen, the at least one stored ultrasound image and to display, in real time, the localization of the micro-device on said at least one ultrasound image.

This approach guarantees a perfect alignment between the localization of the micro-device and the ultrasound image. It thus enables a precise visualization of the micro-device inside the body target part and allows a precise and appropriate control and path planning of said micro-device.

The tracking system may comprises one or several of the following features, taken separately from each other or combined with each other:
- the ultrasound image may be acquired by means of the at least one probe,
- the at least one ultrasound image may be an ULM image
- the at least one probe may comprise at least one ultrasound transducer and the at least one tracker comprises at least one ultrasound sensor,
- the at least one probe may comprise at least one ultrasound sensor and the at least one tracker may comprise at least one ultrasound transducer,
- the at least one tracker may comprise a piezo-electric transducer,
- the micro device may measure between 3µm and 3mm in diameter and up to 2cm in length,
- the localization of the micro-device may reach a precision better than half the size of the wavelength of the ultrasound used to perform the localization,
- the memory of the control unit may be configured to store a succession of ultrasound image of the target body structure, each new ultrasound image replacing the prior one,
- the ultrasound image acquisition may be done in real time, a new ultrasound image acquisition being launched as soon the prior ultrasound image acquisition is terminated, each new ultrasound image replacing the prior one as soon its acquisition is terminated,
- the target body part may be the patient's brain,
- the micro-device may be designed to drop at least one contrast agent inside the target body part, the control unit being able to localize and display, on the ultrasound image, said at least one contrast agent,

The disclosure also relates to a micro-device tracking and localization method implemented by means of the tracking system according to any one of the preceding features, wherein the method may enable, at the same time:
- the real time tracking of the micro-device,
- the real time localization of the micro-device within the internal referential,
- the real time localization of said device inside the target body structure,
the method further enables, at the same time:
- the visualization, on a screen, of an ultrasound image of a target body part of a patient, the ultrasound image being aligned with the internal referential,
- the real time display, on the screen, within the displayed ultrasound image of said micro device localization.

The method may include following steps taken separately from each other or combined with each other:
- the at least one probe may display two working modes:
   o an acquisition mode, during which the at least one probe acquires the ultrasound image, and
   o a tracking mode during which the at least one probe communicates with the at least one tracker,
the at least one probe may be switched from the acquisition mode to the tracking mode at least one time,
- the ultrasound image may be used to:
   - plan at least one micro-device path,
   - monitor, in real time, the micro-device path following,
   - determine, in real time, if an obstacle is situated on the planned path,
   - plan, if needed, a new micro-device path in order to avoid said obstacle.

### BRIEF DESCRIPTION OF THE DRAWINGS

**Figure 1** is an ultrasound image of a target body structure,
**Figure 2** is a schematic view of the tracking system in accordance with the present invention,
**Figure 3** is a schematic view of two trackers fixed on a micro-device in accordance with the present invention,
**Figure 4** is an example of a visualization obtained by means of the tracking system according to the present invention.

### DETAILED DESCRIPTION

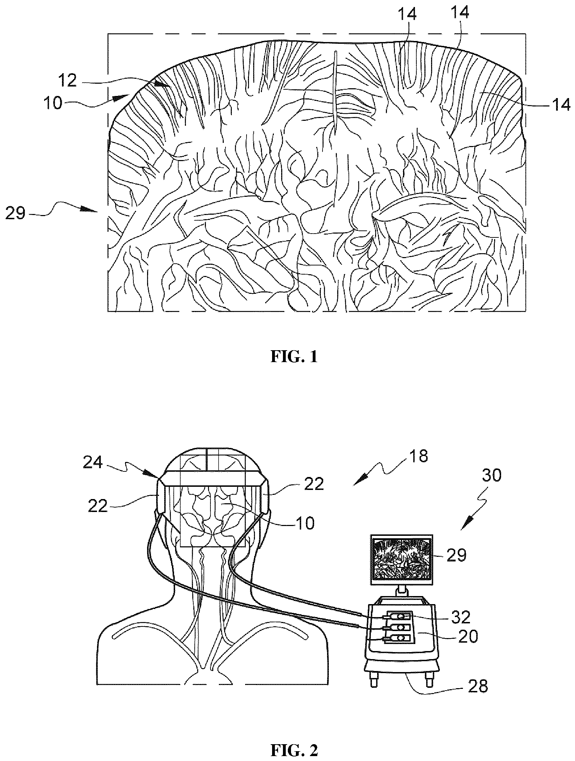

As can be seen on figure 1, a typical target body part 10 like a brain vascular system 12 counts an amazingly high number of blood vessels 14. In order to treat some health issues, like for example tumours, some treatments might imply a micro-device 16, for example a micro-robot, to specifically target a precise point in said target body part 10 to deliver, on this point, a given amount of drug. Such a micro-device 16 usually measures between 3µm and 3mm in diameter and up to 2 cm in length.

In order to be able to work at the micro-device 16 scale, and obtain a precise enough visualization of the micro-device 16 while it is moving inside the target body part 10, it is essential to rely on a very precise remote tracking system, like the tracking system 18 according to the present invention. This remote paradigm imposes high constraints on the volume and energy used by the embedded tracker. In this perspective, a tracking system must answer several strong requirements: sub-millimetric position accuracy, depth from 100 mm and more, real time update (from 20 Hz), non-invasiveness, minimal and at best micro size, at best energetically passive and not harming human body.

In order to achieve this visualization, the tracking system 18 comprises:
- a control unit 20,
- at least one probe 22 configured to be removably secured to a securing body part 24 of the patient, the securing body 24 part surrounding at least partially the target body part 10,
- at least one tracker 26 configured to be connected to the micro-device 16 introduced inside the target body part 10.

In the embodiment illustrated on figure 2, the system 18 includes two probes 22, each secured to a temple of the patient. The securing body part 24 in this embodiment is thus the forehead, and more precisely, the skull, of the patient.

The at least one probe 22 is brought into contact with the securing body part 24. In some embodiments (not represented), the probe 22 is manually handled around the securing body part 24. It is commonly known that, for technical reasons, some gel is spread on the body part 24 and the at least one probe 22. It is nevertheless considered that the at least one probe 22 is brought into contact with the securing body part 24. In some alternative embodiments, the probes 22 are secured to the body part 24 for example by means of a helmet or an elastic holder, as can be seen on figure 2. The probes 22 might also directly be secured to the securing body part 24 by means, for example, of a screwing system. In this case, the probes 22 should be secured surgically to the securing body part 24 of the patient.

Each probe 22 is in constant communication with the control unit 20 on one hand and with the at least one tracker 26 fixed to the micro-device 16 on the other hand. Each probe 22 comprises at least one ultrasound transducer, for example a piezo-electric transducer.

In the current application, the term "transducer" is used synonymously as "emitter" and the term "sensor" is used synonymously as "receptor".

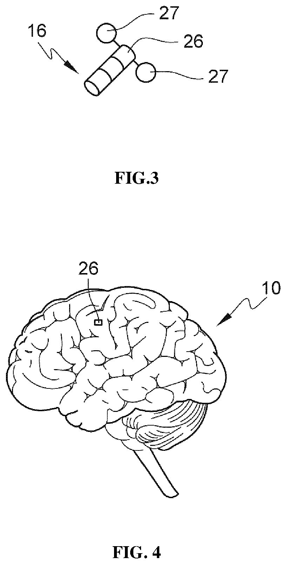

This transducer sends ultrasounds to the tracker 26 on the micro-device 16 inside the target body part 10 (as can be seen on figure 4). In some embodiments, the tracker 26 is a passive tracker and comprises, for example, an encapsulated gas pocket 27 like illustrated on figure 3. In this passive paradigm, the probes 22, (external transducers in the embodiment illustrated on figure 2), secured on the patient's securing body part 24 (in this case, the skull), send ultrasounds inside the target body part 10 (in this case, the human brain). The passive tracker 26 receives the incident waves and scatters them. The waves travel until the securing body part 24 (in this case, the skull) back towards the probe 22. The time of flight between the initial sending and the reception is used to obtain the distance travelled by the waves. Using several probes 22 allows to obtain the 3D position of the tracker 26 relatively to the probes 22.

In some alternative embodiment, the tracker 26 can be an active tracker, actively emitting signals to the probes 22. In those cases, each probe 22 comprises at least one ultrasound sensor and the tracker 26 comprises at least one ultrasound transducer, for example a piezo-electric transducer. The global functioning of the system remains the same, with the ultrasound waves being emitted by the tracker 26 and travelling up to the securing part 24, towards the probes 22.

As already mentioned, in some embodiments, the tracker 26 when being a passive tracker, can comprise at least one encapsulated gas pocket 27 attached to the micro-device 16. This solution is inspired from the principle of ultrasound contrast agents. In this embodiment, each encapsulated gas pocket 27 forms as a very ultrasound reflective object. These encapsulated gas pocket 27 have a large acoustic impedance compared to tissue. This gives them the ability to scatter efficiently the incident ultrasound waves sent by the probes 22 and therefore improve locally the contrast. As object localization precision depends on the signal-to-noise ratio, it allows to track micro-devices 16 smaller than the wavelength deeply inside the target brain part 10, especially the brain, in a non-invasively way. Regarding this embodiment, the tracker 26 comprises several encapsulated gas pockets 27 which are separated by more than half of the detection wavelength are combined to build a full 3D orientation and localization tracker 26. At least two encapsulated gas pocket 27 are needed to obtain the orientation of the micro-device 16.

The control unit 20 further comprises a memory 28 which stores an internal referential R. This internal referential R is defined with reference to the absolute position of each probe 22 with regards to the target body part 10.

The memory 28 further stores at least one ultrasound image 29 of the target body part 10. The ultrasound image 29 can be ULM images or B-mode images, or a Doppler images or an elastography image. All those ultrasound images 29 can be implemented with the same ultrasound probe. The internal referential R enables the co-registration of the ultrasound tracking of the tracker 26 with the ultrasound image 29 acquisition. In some embodiments, the memory also stores at least one pre-established image of the securing body part 24 on which each probe 22 is secured. In those cases, the control unit 20 aligns the at least two images within the internal referential R to precisely position the target body part with respect to each probe 22. In any case, the control unit 20 is able to precisely locate any point of the target body part 10 inside the internal referential R.

The information sensed by each probe 22 is then, in real time, sent to the control unit 20 and the control unit 20 is thus able to localize, in real time, the at least one tracker 26 inside the target body part 10, with regards to the internal referential R.

As already mentioned, the memory 28 is configured to store at least one ultrasound image 29 of the target body structure 10, like for example the image illustrated on figure 1. This ultrasound image can, for example, be an ULM image. The control unit 20 aligns each stored ultrasound image 29 with the internal referential R. This aligned ultrasound image 29 provides a precise 3D mapping of the target body structure 10 of the patient. In case of an ULM image, it provides a very precise 3D mapping of the target body structure 10. This ultrasound image 29 is either obtained prior to the monitoring of the target body part 10 by the system 18, or during the monitoring of the target body structure 10 by the system 18. More particularly, in some embodiments, in order to improve the co-registration, the ultrasound image 29 is performed with the same probe 22 as the one used for tracking and positioning the tracker 26.

In order to reach the desired co-registration, the at least one probe 22 displays two working modes:
- an acquisition mode, during which the at least one probe 22 acquires the ultrasound image 29, and
- a tracking mode during which the at least one probe 22 communicates, by means of ultrasounds with the at least one tracker 26,
the at least one probe 22 is switched from the acquisition mode to the tracking mode at least one time. This enables the system 18 to simultaneously track the micro-device 16 and acquire ultrasound images 29.

In some embodiments, the memory 28 of the control unit 20 can store several ultrasound images 29 of the target body part 10. The memory 28 can thus store a succession of ultrasound image 29 of the target body structure 10. In some embodiments, in order to reduce storage energy, each new ultrasound image 29 replaces the prior one inside the memory 28. In order to increase the precision and accuracy of the mapping of the target body part 10 during its monitoring by the system 18, the ultrasound image acquisition is done in real time. Depending on the ultrasound technique, the ultrasound image 29 acquisition can last several minutes. It it nevertheless considered to be real time acquisition. This provides a real time mapping of the target body part 10 and enables to take quick structure changes into consideration. This real time mapping occurs in that a new ultrasound image 29 acquisition is launched, by the control unit 20, as soon the prior ultrasound image 29 acquisition is terminated, each new ultrasound image 29 thus replacing the prior one as soon its acquisition is terminated. One example could be that the first ultrasound image 29 is an ULM image, further replaced by a Doppler image, which is quicker to acquire.

The control unit 20 is also designed to display, on a screen 30, each acquired and/or stored ultrasound image 29. This is illustrated on figure 2.

By combining the real time ultrasound information obtained from each probe 22 regarding the at least one tracker 26 and the information of the stored ultrasound image 29, the control unit 20 is able to display, in real time, the localization of the at least one tracker 26 on said ultrasound image 29. This enables the surgeon to know, precisely, where the micro-device 16 is.

The control unit 20 further may include a user interface 32 enabling, for example, an operator to indicate, to the micro-device 16 which precise point to reach inside the target body part 10. This user interface may also allow an operator to direct said micro-device 16 in a contactless manner.

The improvement of the micro-device 16 visualization by means of an ultrasound super-resolution technique (like for example the ULM technique) enables a surgeon to precisely monitor the micro-device in a far deeper part of any target body part 10, as for example the brain. Using classic ultrasound imagery enables, for a wave frequency of 1MHz to obtain an image resolution of 0,75mm. Using super-resolution imaging technology enables, for the same frequency, a resolution of 0, 15mm. The super-resolution imaging technology further enables the visualization of little veins which are not visualizable on classic ultrasound imagery. This can help a surgeon to remotely navigate the micro-device 16 around those veins and thus, avoiding to damage or hurt one of those veins and cause internal bleeding.

The micro-device 16 can for example be actuated by either an external engine (for example an external coil, see document PCT/US2019/059096) or an internal engine. It is therefore able to move inside the human body, in any sort of biological medium. The control could be achieved directly with a joystick or through a more complex controller (like a phantom haptic controller) manipulated by a user. The control could also be achieved automatically by following a pre-set pathway. The control signals could be either sent wirelessly or using wires connected to the microdevice.

Using super-resolution ultrasound technology for the tracking further enables the localization of the micro-device 16 to reach a precision better than half the size of the wavelength of the ultrasound used to perform the localization. More particularly, when using ULM technology, one can reach a precision of λ/10 regarding localization and λ/5 regarding visualization.

The ultrasound image 29 thus allows the surgeon to visualize the micro-device 16 and the precise point to be reached by said micro-device 16. Thus, the surgeon can:
- plan at least one path for the micro-device 16 to follow,
- monitor, in real time, the micro-device 16 following said path,
- determine, in real time, if an obstacle is situated on the planned path,
- plan, if needed, a new path for the micro-device 16, in order to avoid said obstacle.

The tracking system 18 according to the invention thus enables to implement a micro-device 16 tracking and localization method, wherein the method enables:
- the real time tracking of the micro-device 16,
- the real time localization of the micro-device 16 within the internal referential (R),
- the real time localization of said device 16 inside the target body structure 10.

The method further allows:
- the visualization, on the screen 30, of at least one ultrasound image 29 of the target body structure 10 of a patient, the ultrasound image 29 being aligned with the internal referential R,
- the real time display, on the screen, 30 within the ultrasound image 29 of said device 16 localization.

If the same probes 22 are used to acquire the ultrasound image 29 and to track the tracker 26, the method thus enables the system 18 to alternate between:
- acquiring an ultrasound image 29,
- tracking the micro-device 16,
without changing the probes 22 and with no need to withdraw the micro-device 16 from the target body part 10 of the patient. This enables an improved real time tracking of the micro-device 16 and an improved real time visualization of the target body part 10.

In order to, for example, inform the surgeon that the drug has been well delivered or to allow a precise monitoring of the target point the micro-device 16 has to reach inside the target body part 10, the micro-device can be designed to drop, at least one contrast agent inside the target body part 10. The contrast agent dropping can be induced by the surgeon or can be pre-programmed. This contrast agent might, for example, be a microbubble. When the at least one contrast agent is dropped inside the target body part 10, it can be sensed by each probe 22 and the control unit 20 is thus able to localize and display, on the ultrasound image 29, said at least one contrast agent, this visualization adds to the precision of the system 18.

## Claims

1. Micro-device (16) tracking and visualization system (18) configured to monitor a target body part (10) of a patient and localizing a micro-device (16) inside said target body part (10), the tracking system (18) comprising:
- a micro-device (16) designed to be remotely steered and controlled from outside the target body part (10),
- a control unit (20) comprising a memory (28), the memory being configured to store at least one ultrasound image (29) of the target body part (10),
- at least one probe (22) configured to be brought in contact with a securing body part (24) of the patient, the securing body part (24) surrounding at least partially the target body part (10),
- at least one tracker (26) configured to be connected to the micro-device (16),
- at least a screen (30),
wherein the at least one probe (22) and the at least one tracker (26) communicate by means of ultrasound technology, the control unit (20) being thus able to localize, in real time, the at least one tracker (26) inside the target body part (10) within an internal referential (R) defined with regards to the at least one probe (22),
wherein the control unit (20) is further designed to display, on the screen (30), the at least one stored ultrasound image (29) and to display, in real time, the localization of the micro-device (16) on said at least one ultrasound image (29).

2. System (18) according to the preceding claim, wherein the ultrasound image (29) is acquired by means of the at least one probe (22).

3. System (18) according to the preceding claim, wherein the at least one ultrasound image (29) is an ULM image.

4. System (18) according to any one of the preceding claims, wherein the at least one probe (22) comprises at least one ultrasound transducer and the at least one tracker (26) comprises at least one ultrasound sensor.

5. System (18) according to claim 1, wherein the at least one probe (22) comprises at least one ultrasound sensor and the at least one tracker (26) comprises at least one ultrasound transducer.

6. System (18) according to the preceding claim, wherein the at least one tracker (26) comprises a piezo-electric transducer.

7. System (18) according to any one of the preceding claims wherein the micro device (16) measures between 3µm and 3mm in diameter and up to 2cm in length.

8. System (18) according to any one of the preceding claims, wherein the localization of the micro-device (16) reaches a precision better than half the size of the wavelength of the ultrasound used to perform the localization.

9. System (18) according to any one of the preceding claims, wherein the memory (28) of the control unit (20) is configured to store a succession of ultrasound image (29) of the target body structure (10), each new ultrasound image (29) replacing the prior one.

10. System (18) according to the preceding claim, wherein the ultrasound image acquisition is done in real time, a new ultrasound image acquisition being launched as soon the prior ultrasound image acquisition is terminated, each new ultrasound image (29) replacing the prior one as soon its acquisition is terminated.

11. System (18) according to any one of the preceding claims, wherein the target body part (10) is the patient's brain.

12. System (18) according to any one of the preceding claims, wherein the micro-device (16) is designed to drop at least one contrast agent inside the target body part (10), the control unit (20) being able to localize and display, on the ultrasound image (29), said at least one contrast agent.

## Patentansprüche

1. Verfolgungs- und Visualisierungssystem (18) einder Mikrovorrichtung (16), konfiguriert, um einen Zielkörperteil (10) eines Patienten zu überwachen und Lokalisieren einer Mikrovorrichtung (16) innerhalb des Zielkörperteils (10), wobei das Verfolgungssystem (18) umfasst:
- eine Mikrovorrichtung (16), die ausgelegt ist, um von außerhalb des Zielkörperteils (10) ferngelenkt und gesteuert zu werden,
- eine Steuereinheit (20), die einen Speicher (28) umfasst, wobei der Speicher konfiguriert ist, um zumindest ein Ultraschallbild (29) des Zielkörperteils (10) zu speichern,
- mindestens eine Sonde (22), konfiguriert, um mit einem sichernden Körperteil (24) des Patienten in Kontakt gebracht zu werden, wobei der sichernde Körperteil (24) den Zielkörperteil (10) mindestens teilweise umgibt,
- mindestens einen Tracker (26), konfiguriert, um mit der Mikrovorrichtung (16) verbunden zu werden,
- mindestens einen Bildschirm (30),
wobei die mindestens eine Sonde (22) und der mindestens eine Tracker (26) mittels Ultraschalltechnologie kommunizieren, wodurch die Steuereinheit (20) in der Lage ist, den mindestens einen Tracker (26) in Echtzeit innerhalb des Zielkörperteils (10) innerhalb einer in Bezug auf die mindestens eine Sonde (22) definierten internen Referenz (R) zu lokalisieren, wobei die Steuereinheit (20) weiter ausgelegt ist, um auf dem Bildschirm (30) das mindestens eine gespeicherte Ultraschallbild (29) anzuzeigen und um in Echtzeit die Lokalisierung der Mikrovorrichtung (16) auf dem wenigstens einen Ultraschallbild (29) anzuzeigen.

2. System (18) nach dem vorstehenden Anspruch, wobei das Ultraschallbild (29) mittels der mindestens einen Sonde (22) erfasst wird.

3. System (18) nach dem vorstehenden Anspruch, wobei das mindestens eine Ultraschallbild (29) ein ULM-Bild ist.

4. System (18) nach irgendeinem der vorstehenden Ansprüche, wobei die mindestens eine Sonde (22) mindestens einen Ultraschallwandler umfasst, und der mindestens eine Tracker (26) mindestens einen Ultraschallsensor umfasst.

5. System (18) nach Anspruch 1, wobei die mindestens eine Sonde (22) mindestens einen Ultraschallsensor umfasst, und der mindestens eine Tracker (26) mindestens einen Ultraschallwandler umfasst.

6. System (18) nach dem vorstehenden Anspruch, wobei der mindestens eine Tracker (26) einen piezoelektrischen Wandler umfasst.

7. System (18) nach einem der vorstehenden Ansprüche, wobei die Mikrovorrichtung (16) zwischen 3µm und 3 mm im Durchmesser und bis zu 2 cm in der Länge misst.

8. System (18) nach irgendeinem der vorstehenden Ansprüche, wobei die Lokalisierung der Mikrovorrichtung (16) eine Präzision erreicht, die besser ist als die halbe Größe der Wellenlänge des Ultraschalls, der verwendet wird, um die Lokalisierung durchzuführen.

9. System (18) nach irgendeinem der vorstehenden Ansprüche, wobei der Speicher (28) der Steuereinheit (20) konfiguriert ist, um eine Ultraschallbildfolge (29) der Zielkörperstruktur (10) zu speichern, wobei jedes neue Ultraschallbild (29) das vorherige ersetzt.

10. System (18) nach dem vorstehenden Anspruch, wobei die Ultraschallbilderfassung in Echtzeit erfolgt, wobei eine neue Ultraschallbilderfassung gestartet wird, sobald die vorherige Ultraschallbilderfassung beendet ist, wobei jedes neue Ultraschallbild (29) das vorherige ersetzt, sobald seine Erfassung beendet ist.

11. System (18) nach irgendeinem der vorstehenden Ansprüche, wobei der Zielkörperteil (10) das Gehirn des Patienten ist.

12. System (18) nach irgendeinem der vorstehenden Ansprüche, wobei die Mikrovorrichtung (16) ausgelegt ist, um mindestens ein Kontrastmittel in den Zielkörperteil (10) einzubringen, wobei die Steuereinheit (20) in der Lage ist, das mindestens eine Kontrastmittel auf dem Ultraschallbild (29) zu lokalisieren und anzuzeigen.

## Revendications

1. Système de suivi et de visualisation (18) d'un micro-dispositif (16) configuré pour surveiller une partie du corps cible (10) d'un patient et localiser un micro-dispositif (16) à l'intérieur de ladite partie du corps cible (10), le système de suivi (18) comprenant :
- un micro-dispositif (16) conçu pour être piloté et commandé à distance depuis l'extérieur de la partie du corps cible (10),
- une unité de commande (20) comprenant une mémoire (28), la mémoire étant configurée pour stocker au moins une image ultrasonore (29) de la partie corporelle cible (10),
- au moins une sonde (22) configurée pour être amenée en contact avec une partie corporelle de fixation (24) du patient, la partie corporelle de fixation (24) entourant au moins partiellement la partie corporelle cible (10),
- au moins un tracker (26) configuré pour être connecté au micro-dispositif (16),
- au moins un écran (30),
dans lequel la au moins une sonde (22) et le au moins un tracker (26) communiquent au moyen d'une technologie ultrasonore, l'unité de commande (20) étant ainsi apte à localiser, en temps réel, le au moins un tracker (26) à l'intérieur de la partie corporelle cible (10) au sein d'un référentiel interne (R) défini par rapport à la au moins une sonde (22),
dans lequel l'unité de commande (20) est en outre conçue pour afficher, sur l'écran (30), la au moins une image ultrasonore (29) mémorisée et pour afficher, en temps réel, la localisation du micro-dispositif (16) sur ladite au moins une image ultrasonore (29).

2. Système (18) selon la revendication précédente, dans lequel l'image ultrasonore (29) est acquise au moyen de la au moins une sonde (22).

3. Système (18) selon la revendication précédente, dans lequel la au moins une image ultrasonore (29) est une image ULM.

4. Système (18) selon l'une quelconque des revendications précédentes, dans lequel la au moins une sonde (22) comprend au moins un transducteur à ultrasons et le au moins un tracker (26) comprend au moins un capteur à ultrasons.

5. Système (18) selon la revendication 1, dans lequel la au moins une sonde (22) comprend au moins un capteur à ultrasons et le au moins un tracker (26) comprend au moins un transducteur à ultrasons.

6. Système (18) selon la revendication précédente, dans lequel le au moins un tracker (26) comprend un transducteur piézoélectrique.

7. Système (18) selon l'une quelconque des revendications précédentes dans lequel le micro-dispositif (16) mesure entre 3µm et 3 mm de diamètre et jusqu'à 2 cm de longueur.

8. Système (18) selon l'une quelconque des revendications précédentes, dans lequel la localisation du micro-dispositif (16) atteint une précision meilleure que la moitié de la taille de la longueur d'onde de l'ultrason utilisé pour effectuer la localisation.

9. Système (18) selon l'une quelconque des revendications précédentes, dans lequel la mémoire (28) de l'unité de commande (20) est configurée pour stocker une succession d'images ultrasonores (29) de la structure corporelle cible (10), chaque nouvelle image ultrasonore (29) remplaçant la précédente.

10. Système (18) selon la revendication précédente, dans lequel l'acquisition d'image ultrasonore est réalisée en temps réel, une nouvelle acquisition d'image ultrasonore étant lancée dès que l'acquisition d'image ultrasonore précédente est terminée, chaque nouvelle image ultrasonore (29) remplaçant la précédente dès que son acquisition est terminée.

11. Système (18) selon l'une quelconque des revendications précédentes, dans lequel la partie du corps cible (10) est le cerveau du patient.

12. Système (18) selon l'une quelconque des revendications précédentes, dans lequel le micro-dispositif (16) est conçu pour déposer au moins un agent de contraste à l'intérieur de la partie corporelle cible (10), l'unité de commande (20) étant apte à localiser et afficher, sur l'image échographique (29), ledit au moins un agent de contraste.
